Europäisches Patentamt

European Patent Office

Office européen des brevets

(11) Publication number: **0 285 805 B1**

# EUROPEAN PATENT SPECIFICATION

(45) Date of publication of patent specification: **14.10.92**

(51) Int. Cl.⁵: **A61K 31/71**, A61K 35/74, C07G 11/00, C12P 1/04

(21) Application number: **88103207.2**

(22) Date of filing: **02.03.88**

(54) **New heptaene antibiotic, V-28-3M.**

(30) Priority: **06.03.87 JP 51570/87**

(43) Date of publication of application:
**12.10.88 Bulletin 88/41**

(45) Publication of the grant of the patent:
**14.10.92 Bulletin 92/42**

(84) Designated Contracting States:
**DE FR GB IT**

(56) References cited:

**PATENT ABSTRACTS OF JAPAN vol. 11, no. 13 (C-397)(2460), 14 January 1987.**

**CHEMICAL ABSTRACTS, vol. 86, no. 15, 11 April 1977, Columbus, OH (US); W.CHEN et al., p. 24, no. 100832s.**

(73) Proprietor: **AJINOMOTO CO., INC.**
**5-8, Kyobashi 1-chome, Chuo-ku Tokyo 104(JP)**

(72) Inventor: **Komoda, Yasuo**
**3-1, Toyama Shinjyuku-ku Tokyo(JP)**

Inventor: **Yokogawa, Yasunori**
**No. 1-1, Suzuki-cho Kawasaki-ku Kawasaki-shi Kanagawa-ken(JP)**
Inventor: **Okunishi, Masahiko**
**No. 1-1, Suzuki-cho Kawasaki-ku Kawasaki-shi Kanagawa-ken(JP)**
Inventor: **Ishii, Koichi**
**No. 1-1, Suzuki-cho Kawasaki-ku Kawasaki-shi Kanagawa-ken(JP)**
Inventor: **Shibai, Hiroshiro**
**No. 1-1, Suzuki-cho Kawasaki-ku Kawasaki-shi Kanagawa-ken(JP)**
Inventor: **Noumi, Ryosaku**
**12-8, Ando 4-chome Asaminami-ku Hiroshima-shi Hiroshima-ken(JP)**

(74) Representative: **Strehl, Schübel-Hopf, Groening**
**Maximilianstrasse 54 Postfach 22 14 55 W-8000 München 22(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to a new heptaene compound V-28-3M. This substance is effective as an antibiotic.

Recently, the treatment of bacterial infections has been greatly improved by the use of antibiotics and many other chemotherapeutic agents. The use of these chemotherapeutic agents, however, has induced a difficult problem, i.e. profound mycosis caused by the infection with Eumycetes.

The applicant has already disclosed a new heptaene antibiotic which exhibits antimycotic activity, in JA-A-61-189224. While this substance exhibits intense antimycotic activity, it is highly zootoxic, so that it requires further improvement for use as an antibiotic agent.

The problem to be solved by the present invention is to obtain a new antimycotic agent which has higher antimicrobial activity and exhibits less toxicity.

The inventors have synthesized a derivative of the heptaene antibotic V-28-3 (hereinafter referred to as V-28-3) for the purpose of obtaining an antimycotic agent with a high degree of antimicrobial activity towards Eumycetes and with a lower level of toxicity. As a result, it could be found that the methyl ester of V-28-3 (hereinafter referred to as V-28-3M) is a new heptaene derivative with the intended higher degree of antimicrobial activity towards Eumycetes and a zooblast toxicity lower than that of V-28-3.

The compound V-28-3M thus obtained gives a single peak at 7.5 minutes in high performance liquid chromatography (hereinfafter referred to as HPLC, 1.0 ml/minute flow rate) using a Novapack® C18 carrier (Waters), a 10 mM ammonium acetate buffer solution containing 65% acetonitrile as a mobile phase, and a UV detector (405 nm and 254 nm), and exhibits the following physicochemical properties:

1) Elementary analysis (%): C: 58.95, H: 7.64, N: 2.39

2) Molecular weight (FABMS, $(M+H)^+$): 1127

3) Specific rotation: $[\alpha]_D^{20}$ + 296° (C = 0.1% DMSO)

4) Melting behaviour: It turns brown at 135°C.

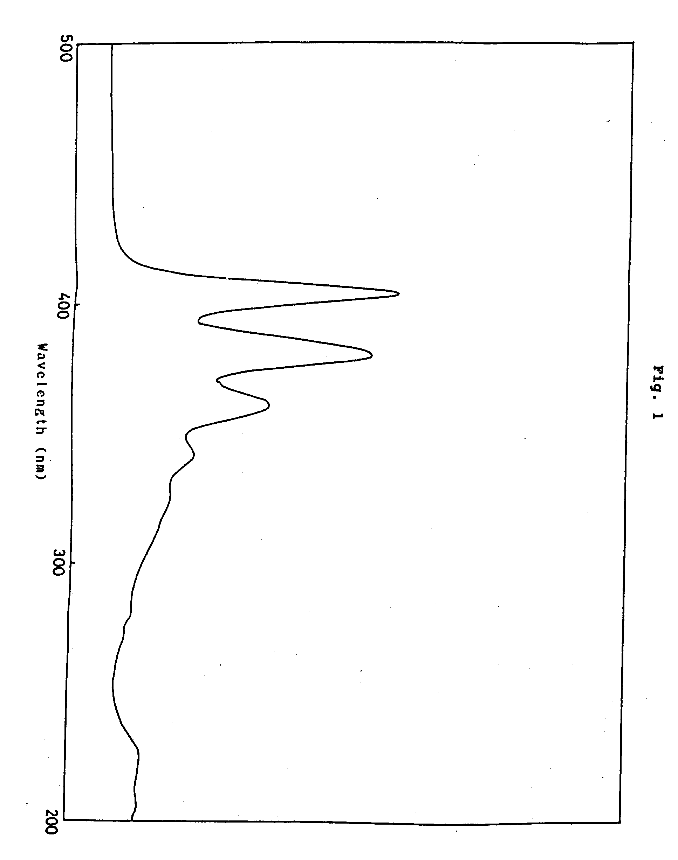

5) UV spectrum: As shown in Fig. 1.

6) Solubility in solvents:

It is insoluble in water, diethyl ether, hexane and chloroform.

It is slightly soluble in methanol, ethanol and n-butanol.

It is soluble in dimethyl sulfoxide and N,N-dimethylformamide.

7) Color reaction: Phenol-sulfuric acid: positive; ferric chloride: negative; ninhydrine: positive; concentrated sulfuric acid: positive; iodine: positive.

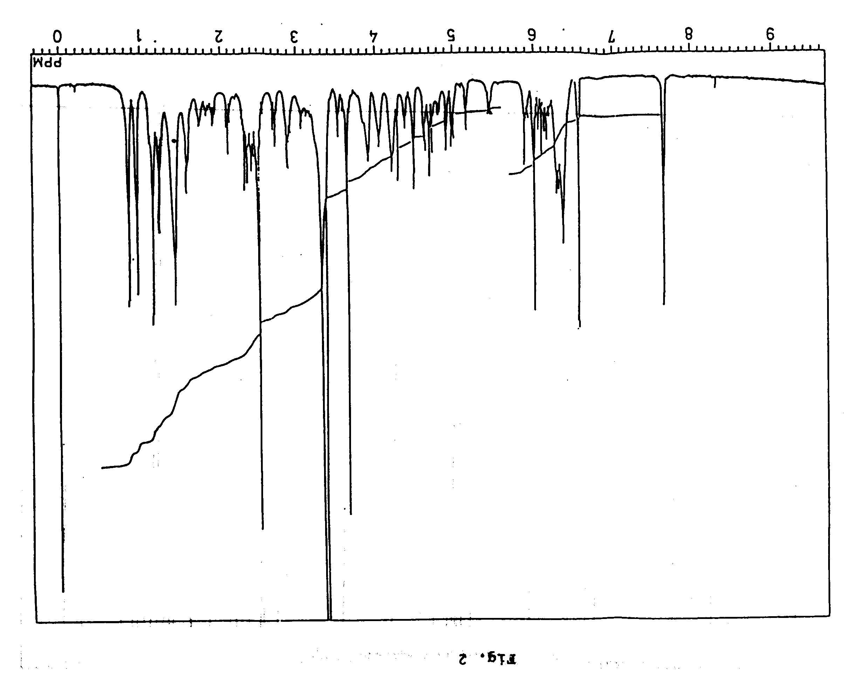

8) $^1$H-NMR spectrum: As shown in Fig. 2.

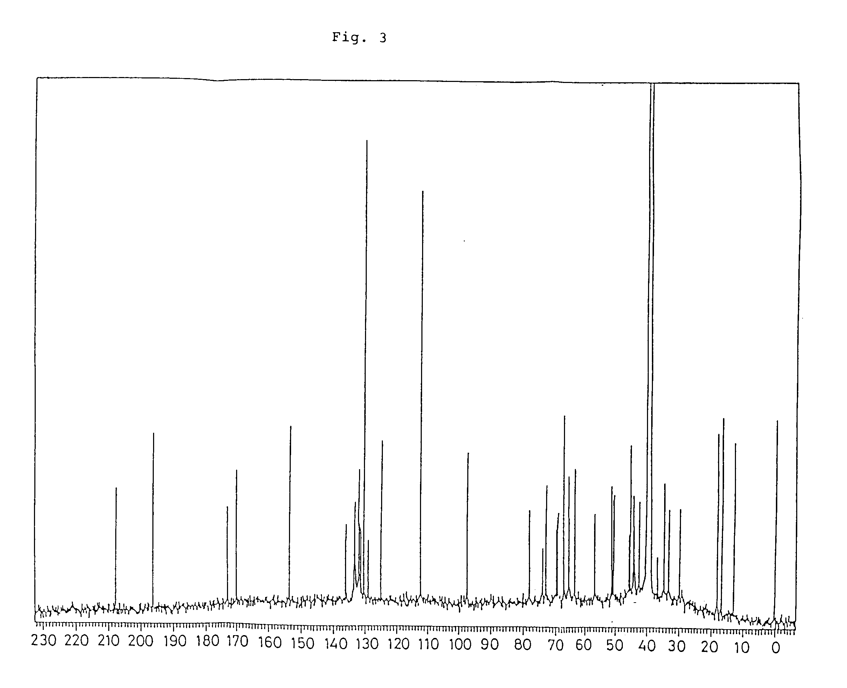

9) $^{13}$C-NMR spectrum: As shown in Fig. 3.

10) UV MeOH λmax. ($\epsilon$): 405 (85,510), 382 (77,551)

362 (46,531), 344 (25,714).

V-28-3M can be produced by subjecting V-28-3 to any usual method of methyl esterification reaction. An example of the production process is given below. V-28-3M can be produced by adding a diazomethane solution prepared by the usual method to a methyl alcohol suspension of V-28-3 at ice temperature, and stirring the solution at room temperature for about 30 minutes.

V-28-3 as the starting compound can be produced according to a method described in Japanese patent publication 61-189224 (JA-A-61-189224).

The V-28-3M thus produced by a reaction can be separated and purified by methods which are generally used for the isolation of antibiotics from the residue after concentrating the reaction liquid under reduced pressure.

The compound V-28-3M is a potent antimycotic and antibacterial agent.

As shown in Table 1, the minimal inhibitory concentration (MIC) of V-28-3M to Candida albicans ATCC 10231 is lower than the MIC of amphotericin B and V-28-3. Further, the compound displayed strong antimicrobial activity.

Table 1


Minimal inhibitory concentration (MIC, μg/ml)


| Strain | V-28-3M | V-28-3 | amphotericin B |
|---|---|---|---|
| Candida albicans (ATCC 10231) | 0.39 | 0.78 | 1.56 |


The experiment (Table 1) was performed by the usual measuring method for MIC using Sabraud's agar, and the MIC was determined 48 hours later.

The 50 % growth inhibitory concentration of 28-3M to L1210 cells, as listed in Table 2, is about 6 times higher than that of V-28-3, which shows, that the cytotoxicity is low.


Table 2

Cytotoxicity (50% growth inhibitory concentration, μg/ml)


| Cell name | V-28-3M | V-28-3 |
|---|---|---|
| L1210 | 1.4 | 0.23 |


RPMI 1640 culture medium was used for the experiment (Table 2), which was conducted by the usual cell growth inhibitory measuring method. The cytotoxicity was determined 48 hours later.

As shown in Table 1, V-28-3M displays strong antimicrobial activity towards Candida albicans, and as shown in Table 2, it has a low level of toxicity and can be used as a new antimycotic agent.

The present invention is described in detail by the following example and the drawings, where

Figure 1 represents the UV spectrum of V-28-3M measured in methyl alcohol, showing the intensity of absorption on the longitudinal axis and wavelength (nm) on the transverse axis.

Figure 2 and 3 represent $^1$H-NMR and $^{13}$C-NMR spectra, measured at 400 MH and 100 MH respectively. The longitudinal axis shows the intensity of absorption and the transverse axis a chemical shift expressed in ppm based on TMS.


Example


A 100 ml culture medium of the composition shown in Table 3 was poured into a 500 ml flask, and was sterilized by heating at 120°C for 10 minutes. By using a platinum loop, this medium was inoculated with Streptomyces arenae V-28-3 (FERM P-8099) grown on an agar slant of the same composition, and cultured at 27°C for 3 days while being agitated.

The strain identified above as FERM P-8099 was originally deposited on February 16, 1985 at the Fermentation Research Institute, Agency of Industrial and Technology, Ministry of International Trade and Industry (FRI), 1-3, Higashi 1-chome, Yatabe-machi, Tsukuba-gun, Ibaragi-ken 305, Japan. The deposit was then converted into a deposit under the Budapest Treaty on October 27, 1987, and was given the accession number FERM BP = 1537.

## Table 3
### Composition of the culture medium*

| Components | Content (in 1.0 l) |
|---|---|
| Soluble starch | 30.0 g |
| Malt extract | 6.0 g |
| Yeast extract | 6.0 g |
| Polypeptone | 12.0 g |
| Table salt | 5.0 g |
| $MgSO_4 \cdot 7H_2O$ | 0.5 g |

\* (pH: 7.0)

20 liter of the culture medium, to which silicon KM-75 (10 ml) as an antifoaming agent had been further added, was placed into a 30 l jar fermenter, and sterilized by heating at 120°C for 10 minutes. The solution was then inoculated with the above culture medium, and aerobic culture was performed at 27°C for 24 hours while stirring. (Aeration amount 1/2 V.V.M; stirring revolution 200 rpm.) The culture solution was centrifuged (4,000 rpm for 10 minutes), and a 1.6 kg microbial cake was obtained. 1.5 l of 80% methanol was added to a 500 g cell cake of the 1.6 kg cake, and ammonia water was added to change the pH to 9.5. It was then stirred slowly, and the extraction operation was continued for 3 hours at room temperature. The solution was centrifuged to remove insoluble matter and to obtain the extract. The pH of the extract solution was lowered to 7.0, and 1.5 l of water was added. Then the solution was passed through a DIAION® HP-20 adsorption carrier column (10 × 30 cm) (manufactured by Mitsubishi Chemical Industries). After washing this column with a small quantity of 50% methanol, elution fractions were eluted with 80% methanol containing 0.45% ammonia water. A fraction which has antimicrobial activity towards Candida albicans HUT 7501 was collected from the elution fractions and concentrated under reduced pressure to obtain a yellowish brown precipitate by centrifugation. By washing this precipitate with cold acetone, and drying it under reduced pressure, 600 mg of yellowish green powder was obtained. This powder was suspended in 100 ml chloroform solution and passed through a high flow super cell column (5 × 20 cm). After washing the column with 100 ml chloroform and then with a 100 ml chloroform-methanol mixture (1:1 mixing ratio), elution fractions were eluted with acetonitrile - 0.05 M ammonium acetate (pH 9.5) eluent (1:1 mixing ratio). An active fraction was collected from the elution fractions, and the solvent was removed under reduced pressure. The precipitate produced was collected by centrifugation and washed with acetone and dried under reduced pressure. Thus 420 mg of yellow powder was obtained. 60 mg of the powder was dissolved in 5.0 ml of a 66% tetrahydrofuran solution. This solution was passed through an LRP-1 column (Whatmann C-18 reversed phase chromatography carrier), and active antimicrobial fractions were eluted with an eluent of the composition acetonitrile - 0.05 M ammonium acetate (pH 9.0) (4.5 : 5.5 mixing ratio). The antimicrobial substance was separated by elution in two fractions. (The active substance eluted first was identified as Partricin B by its $^1$H-NMR spectrum.) The antimicrobial active fraction eluted later was collected and the solvent slowly removed under reduced pressure conditions until a whitish turbidity was observed. The fraction was preserved at 5°C overnight to precipitate yellow crystals. The yellow crystals were collected by centrifugation and washed with cold water and cold acetone, and then dried under reduced pressure. In this way, 30 mg of V-28-3 was obtained.

The V-28-3 (20 mg) thus obtained was suspended in 4 ml methyl alcohol. A tetrahydrofuran solution of diazomethane was then dropped into the ice-cold suspension. After stirring the reactant liquid at room temperature for 30 minutes, nitrogen gas was blown into the reactant liquid to remove the solvent. 21 mg of dry solid matter was obtained. The dry solid matter was dissolved in the lower layer liquid of a chloroform-methyl alcohol-water mixture (2:2:1 mixing ratio) and subjected to countercurrent droplet distribution chromatography with the lower layer liquid as a stationary phase and the upper layer liquid as a mobile phase. By means of the HPLC mentioned before, the eluate was examined, and fractions containing only V-28-3M were concentrated and dried. As a result, V-28-3M (15 mg) was separated as a yellow powder. The results of elementary analysis of the yellow powder were C: 58.95 %, H: 7.64%, and N: 2.39 %. UV spectrum, $^1$H-NMR spectrum and $^{13}$C-NMR spectrum are shown in Figs. 1, 2 and 3 respectively.

## Claims

1.  New heptaene V-28-3M having the following physicochemical properties:
    a) Material property: yellow powder
    b) Elementary analysis: C: 58.95, H: 7.64, N: 2.39
    c) Molecular weight (FABMS, $(M + H)^+$) : 1127
    d) Molecular formula: $C_{59}H_{86}N_2O_{19}$
    e) Specific rotation: $[\alpha]_D^{20}$ + 296°C (0.1% DMSO)
    f) Melting behaviour: It turns brown at 135°C.
    g) UV spectrum: As shown in Fig. 1
    h) Solubility in solvents:
    Insoluble in water, diethyl ether, hexane and chloroform;
    slightly soluble in methanol, ethanol and n-butanol;
    soluble in dimethyl sulfoxide and N,N-dimethyl-formamide.
    i) Color reaction: phenol-sulfuric acid: positive; ferric chloride: negative; ninhydrine: positive; concentrated sulfuric acid: positive; iodine: positive.
    j) $^1$H-NMR spectrum: As shown in Fig. 2.
    k) $^{13}$C-NMR spectrum: As shown in Fig. 3.

## Patentansprüche

1.  Neues Heptaen V-28-3M mit den folgenden physikochemischen Eigenschaften :
    a) Stoffeigenschaft : gelbes Pulver
    b) Elementaranalyse : C: 58,95 %, H: 7,64 %, N: 2,39 %
    c) Molekulargewicht : (FABMS, $(M + H)^+$) : 1127
    d) Molekülformel : $C_{59}H_{86}N_2O_{19}$
    e) Spezifische Drehung : $[\alpha]_D^{20}$ + 296°C (0,1 % DMSO)
    f) Schmelzverhalten : Es wird braun bei 135°C.
    g) UV-Spektrum : gemäß Fig. 1
    h) Löslichkeit in Lösungsmitteln :
    Unlöslich in Wasser, Diethylether, Hexan und Chloroform;
    geringfügig löslich in Methanol, Ethanol und n-Butanol;
    löslich in Dimethylsulfoxid und N,N-Dimethylformamid.
    i) Farbreaktion : Phenol-Schwefelsäure : positiv; Eisen(III)chlorid : negativ; Ninhydrin : positiv; konzentrierte Schwefelsäure : positiv; Jod : positiv.
    j) $^1$H-NMR Spektrum : gemäß Fig. 2.
    k) $^{13}$C-NMR Spektrum : gemäß Fig. 3.

## Revendications

1.  Nouvel heptaène V-28-3M ayant les propriétés physicochimiques suivantes :
    a) Propriété du matériau : poudre jaune
    b) Analyse élémentaire (%) : C: 58,95, H: 7,64, N: 2,39
    c) Masse moléculaire (spectrométrie de masse FABMS, $(M+H)^+$) : 1127
    d) Formule moléculaire : $C_{59}H_{86}N_2O_{19}$
    e) Rotation spécifique : $[\alpha]_D^{20}$ +296°C (0,1% DMSO)
    f) Comportement à la fusion : devient brun à 135°C.
    g) Spectre UV : comme présenté dans la figure 1.
    h) Solubilité dans les solvants :
    insoluble dans l'eau, l'éther diéthylique, l'hexane et le chloroforme ;
    légèrement soluble dans le méthanol, l'éthanol et le n-butanol ;
    soluble dans le diméthylsulfoxyde et le N,N-diméthylformamide.
    i) Réactions colorées : acide phénolsulfurique : positive ; chlorure ferrique : négative ; ninhydrine :

positive ; acide sulfurique concentré : positive ; iode : positive.

j)Spectre de RMN de $H^1$ : comme représenté dans la figure 2.

k)Spectre de RMN de $C^{13}$ : comme représenté dans la figure 3.

Fig. 1

Fig. 2

EP 0 285 805 B1

Fig. 3